# EUROPEAN PATENT SPECIFICATION

(11) **EP 1 178 850 B1**
(45) Date of publication and mention of the grant of the patent: **29.10.2003**
(21) Application number: 00936149.4
(22) Date of filing: 19.05.2000
(51) Int. Cl.: A61L 31/08

(54) **ANTIMICROBIAL ARTICLES**
ANTIMIKROBIELLE GEGENSTÄNDE
ARTICLES ANTIMICROBIENS

(30) Priority: 21.05.1999 US 135271 P; 17.05.2000 US 572549
(43) Date of publication of application: 13.02.2002
(73) Proprietor: 3M Innovative Properties Company, Saint Paul, MN 55133-3427 (US)
(72) Inventor: ANDREWS, Jeffrey, F., Saint Paul, MN 55133-3427 (US); SCHOLZ, Matt, T., Saint Paul, MN 55133-3427 (US); TUCKER, Joseph, A., Saint Paul, MN 55133-3427 (US); POURNOOR, Kaveh, Saint Paul, MN 55133-3427 (US)
(74) Representative: VOSSIUS & PARTNER
(86) International application number: US0013974
(87) International publication number: WO00071183

(56) References cited:
- WO-A-97/11912
- US-A- 5 882 357
- KABARA J J ET AL: "ANTIMICROBIAL LIPIDS: NATURAL AND SYNTHETIC FATTY ACIDS AND MONOGLYCERIDES" LIPIDS,US,CHAMPAIGN, IL, vol. 12, no. 9, 1 September 1977 (1977-09-01), pages 753-759, XP000563038 ISSN: 0024-4201

## Description

The invention relates to articles that include an antimicrobial composition and to methods of making such articles. In particular, the invention relates to coated articles that include a fatty acid monoester and an enhancer said enhancer being a chelating agent or an organic acid.

Antimicrobial nonwoven articles and other types of extruded materials have been prepared by incorporation of antimicrobial agents directly into a polymeric hot melt prior to extrusion. This method allows the antimicrobial agents to be directly incorporated into the nonwoven and migrate onto the surface. Durable antimicrobial articles are obtained from such processes due to the continuous migration over time of the antimicrobial to the surface of the article. Hot-melt processes, however, require very high temperatures, e.g., 300°C or higher. At such temperatures, many antimicrobial agents, especially organic molecules, face problems with thermostability and volatility. Thus, alternative methods for incorporating antimicrobial agents into nonwoven materials are needed.

Articles having a coating of a fatty acid monoester to avoid bacterial adhesion to a surface thereon are reported in International Publication No. WO 97/11912, published April 3, 1997. Such articles include a submergible surface such as a boat hull, marine structure, or a surface within an aqueous system such as a pump or heat exchanger. The submergible surface is made to inhibit the adherence of bacteria by contacting the surface with a polyglycol fatty acid ester or a mixture of polyglycol fatty acid esters.

Textile articles to which a heterocyclic N-halamine is attached as an antibacterial agent are reported in U.S. Pat. No. 5,882,357, issued March 16, 1999. The heterocyclic N-halamine is covalently attached to the textile article using a wet finishing process. The antibacterial activity of the article may be regenerated by washing the article with a halogenated solution.

Natural and synthetic lipophilic compounds having antibacterial activity against Gram positive and Gram negative bacteria are reported in Kabara *et al*., Lipids, vol. 12, no. 9, pp.753-759 (1977). Unsaturated fatty acids, selena-substituted fatty acids, and monoglycerides are reported to have measurable antimicrobial activity.

The invention is based on the discovery that synergistic combinations of fatty acid monoesters and enhancers wherein the enhancer is a chelating agent or an organic acid have been found to have antimicrobial activity on dry or essentially dry articles. Articles that are coated with compositions containing such molecules arid dried are self-disinfecting, i.e., microorganisms that come into contact with the surface of the article are killed. Fatty acid monoesters and enhancers useful in the invention generally are of low mammalian toxicity, allowing articles of the invention to be used in a wide variety of applications, including applications resulting in direct contact with food or direct contact with humans and animals.

In one aspect, the invention features an antimicrobial article including a coating. The coating includes a fatty acid monoester and an enhancer, wherein the fatty acid monoester and the enhancer are present in an amount effective to kill at least 99.9% of microorganisms, e.g., Gram positive or Gram negative bacteria, or viruses. In some embodiments, the fatty acid monoester and the enhancer are present in an amount effective to kill at least 99.99% of microorganisms. The fatty acid monoester can be glycerol monolaurate, glycerol monocaprylate, glycerol monocaprate, propylene glycol monolaurate, propylene glycol monocaprylate, or propylene glycol monocaprate. The enhancer is selected from a chelating agent, e.g., sodium acid pyrophosphate, acidic sodium hexametaphosphate, or ethylenediaminetetraacetic acid and salts thereof, or an organic acid, e.g., an organic acid selected from the group consisting of lactic acid, tartaric acid, adipic acid, succinic acid, citric acid, ascorbic acid, malic acid, mandelic acid, acetic acid, sorbic acid, benzoic acid, and salicylic acid. Lactic acid and mandelic acid are particularly useful enhancers. The coating further can include a surfactant.

The antimicrobial article can be a nonwoven material, a woven fabric or web material, a sponge material, a fibrous batt material, a foam material, a surgical drape or gown, a face mask, a wound dressing, a disposable diaper, filter media, a cast padding, a stockinette, a respirator, food packaging, dental floss, a sponge, a textile, or a wipe. The antimicrobial article can have increased hydrophilicity as compared with a corresponding article without the coating.

The invention also features a surgical drape or face mask that includes a coating, wherein the coating includes a fatty acid monoester and an enhancer, and wherein the fatty acid monoester and the enhancer are present in an amount effective to kill at least 99.9% of microorganisms. The coating can further include a surfactant.

In another aspect, the invention features a method of making an antimicrobial article. The method includes treating the article with an antimicrobial composition, wherein the composition includes 0.001 wt. % to 30 wt. % of a fatty acid monoester and 0.001 wt. % to 85 wt. % of an enhancer; and drying the treated article. The antimicrobial article is effective for killing at least 99.9% of microorganisms. The fatty acid monoester can preferably be present at 0.01 wt. % to 5.0 wt. % in the antimicrobial composition. The enhancer can preferably be present at 0.5 wt. % to 5.0 wt. % in the antimicrobial composition. The antimicrobial composition further can include 0.001 wt. % to 30 wt. % of a surfactant, e.g., 0.5 wt. % to 5.0 wt. % of the antimicrobial composition.

The invention also features an alternative method of making an antimicrobial article. The method includes treating the article with a first composition comprising 0.001 wt. % to 30 wt. % of a fatty acid monoester; treating the article with a second composition comprising 0.001 wt. % to 85 wt. % of an enhancer; and drying the treated article, wherein the antimicrobial article is effective for killing at least 99.9% of microorganisms. The fatty acid monoester can preferably be present at 0.01 wt. % to 5.0 wt. % in the first composition. The enhancer can preferably be present at 0.5 wt. % to 5.0 wt. % in the second composition. The first composition further can include 0.001 wt. % to 30 wt. % of a surfactant, e.g., 0.5 wt. % to 5.0 wt. % of the first composition.

In the present invention, selected articles are coated with liquid compositions containing a fatty acid monoester and an enhancer. After drying, it has been discovered, surprisingly, that the articles possess synergistic antimicrobial activity. Dry or essentially dry articles of the invention reduce the bacterial load of a challenge by greater than 99.9%. In certain cases, the bacterial load of both Gram-positive and Gram-negative bacteria are reduced by 99.9%. In contrast, identical articles coated with liquid compositions containing only fatty acid monoester or an enhancer typically fail to reduce bacterial load by 99.9%, and in particular, fail to reduce Gram-positive and Gram-negative bacteria load by 99.9%.

Thus, antimicrobial articles of the invention include a coating having a fatty acid monoester and an enhancer present in an amount effective to kill at least 99.9% of microorganisms upon contact with the article. In some embodiments, at least 99.99% of microorganisms are killed. As used herein, "antimicrobial" or "antimicrobial activity" means that an article has antimicrobial activity as measured by the American Association of Textile and Color Chemists (AATCC) Test Method 100-1993 (AATCC Technical Manual, 1997, pp. 143 to 144). This test method involves exposing treated articles of the present invention at selected times and temperatures to known or readily available viable bacterial strains, such as *Klebsiella pneumonia, Escherichia coli, Staphylococcus aureus,* and *Staphylococcus epidermidis* in a culture medium. Typically, treated articles are exposed for about 24 hours at temperatures of about 22°C to about 35°C. After a specific exposure time, treated and untreated control samples are neutralized and the number of bacteria are determined by standard microbiological techniques. By comparing the number of bacteria from the treated sample to the number of bacteria from the control (treated samples at time zero or an untreated sample), the percent reduction of bacteria attributable to the antibacterial treatment can be calculated.

As used herein "amount effective" means that the amounts of fatty acid monoester and enhancer, as a whole, provide a spectrum of antimicrobial activity sufficient to kill most microorganisms on the article. The antimicrobial activity preferably extends to viruses, fungi, and bacteria, including Gram-positive and Gram-negative bacteria, pathogenic or undesired bacteria such as bacteria known to cause or be associated with food poisoning in humans, and bacteria related to or associated with food spoilage. The concentrations or amounts of each of the components, when considered separately, do not kill as great a spectrum of pathogenic or undesired microorganisms or reduce the number of such microorganisms to an acceptable level. Thus, the components of the composition when used together provide a synergistic antimicrobial activity to the article when compared to the same components used alone and under the same conditions.

The capability to reduce a bacterial load preferably is maintained for a sustained period of exposure. For example, the antimicrobial articles of the present invention can have a shelf life of at least about one year, and, more preferably, of at least about two years. Without being bound by a particular mechanism, it is thought that the dry state of the article prevents reaction of the fatty acid monoester with other components. Liquid compositions of fatty acid monoesters and enhancers possess synergistic antimicrobial activity. Fatty acid monoesters, however, are inherently reactive, especially in the presence of enhancers such as organic acids or chelating agents. For example, the monoesters can hydrolyze in an aqueous medium to the corresponding fatty acid, transesterify with a hydroxy-containing enhancer (e.g., lactic acid), or transesterify with a hydroxy-containing solvent. As a result of these reactions, the antimicrobial activity of the liquid composition may be reduced and shelf life may be shortened to less than one year. As most articles of the invention are dry when packaged or stored, the articles are not in intimate contact with an aqueous liquid or other solvent vehicle. Consequently, the fatty acid monoester is much less subject to reactions such as hydrolysis or transesterification over time.

### Fatty acid monoesters and Enhancers

Fatty acid monoesters suitable for use in the invention are monoesters that generally are considered food grade, are generally recognized as safe (GRAS), and/or are FDA-cleared food additives. In particular, fatty acid monoesters derived from C₈ to C₁₂ fatty acids such as glycerol monoesters of lauric, caprylic, or capric acid and/or propylene glycol monoesters of lauric, caprylic, or capric acid are useful. Monoglycerides useful in the invention typically are available in the form of mixtures of unreacted glycerol, monoglycerides, diglycerides, and triglycerides. However, it is preferred to use materials that contain a high concentration (e.g., greater than about 85 wt. %, preferably greater than about 90 wt. %, and more preferably greater than about 92%) of monoglyceride. Examples of particularly useful commercially available materials include glycerol monolaurate (GML), available from Med-Chem Laboratories, East Lansing, MI, under the tradename LAURICIDIN™, glycerol monocaprylate (GM-C8) and glycerol monocaprate (GM-C10) available from Riken Vitamin Ltd., Tokyo, Japan under the tradenames POEM™ M-100 and POEM™ M-200, respectively, and those available from the Henkel Corp. of Germany under the tradename "MONOMULS™ 90 L-12". Propylene glycol monocaprylate (PG-C8) and propylene glycol monocaprate (PG-C10) are available from Uniquema International, Chicago, IL.

Suitable enhancers are organic acids and chelating agents. Preferably, the enhancers are food grade, GRAS approved, and/or FDA-cleared food additives. Organic acids can include, for example, lactic acid, tartaric acid, adipic acid, succinic acid, citric acid, ascorbic acid, malic acid, mandelic acid, acetic acid, sorbic acid, benzoic acid, and salicylic acid. Chelating agents can include, for example, sodium acid pyrophosphate, acidic sodium hexametaphosphate (such as SPORIX™ acidic sodium hexametaphosphate), and ethylenediaminetetraacetic acid (EDTA) and salts thereof. Lactic acid and mandelic acid are particularly useful.

### Preparing Articles of the Invention

Fatty acid monoester, enhancer, and other acceptable materials (e.g., emulsifiers) are dissolved, dispersed, and/or emulsified in a liquid vehicle such as water, alcohol, or propylene glycol to form a liquid composition. Non-limiting examples of emulsifiers include anionic and nonionic surfactants such as dioctyl sodium sulfosuccinate, sodium lauryl sulfate, acyl lactylates such as sodium lauroyl lactylate, sorbitan esters such as sorbitan monolaurate, dodecylbenzene sulfonate and its salts, polyglycerol esters such as decaglyceryl tetraoleate, and block copolymers of polyalkylene oxide, e.g., polyethylene oxide and polypropylene oxide, available as Pluronics™ and Tetronics™ from BASF. Dioctyl sodium sulfosuccinate is commercially available as GEMTEX™ SC40 surfactant (40% dioctyl sodium sulfosuccinate in isopropanol) from Finetex Inc., Spencer, North Carolina.

Typically, the fatty acid monoester is about 0.001 to 30 weight % and the enhancer is about 0.001 to 85 wt. % of the liquid composition used to prepare the article of the invention. For example, the fatty acid monoester can be 0.01 to 5.0 wt. % of the liquid composition and the enhancer can be about 0.5 to 5.0 wt. % of the liquid composition. Surfactants, when present, typically are 0.001 to 30 wt. % of the liquid composition and preferably, 0.5 wt. % to 5.0 wt. % of the liquid composition. The liquid composition may also include a liquid carrier or solvent, e.g., water or an alcohol. The liquid composition is coated on the article by applying the composition to a portion of or over the entire exterior surface of the article using conventional application techniques, such as dipping, spraying, printing, brushing, sponging, or padding at temperatures preferably ranging from about 10°C to about 100°C. Alternatively, the article may be prepared by treating with a first liquid composition containing the fatty acid monoester and optionally a surfactant, and separately treated with a second liquid composition containing the enhancer. Preferably, the coated surface or surface portion of the article is fully wetted with the liquid composition. The liquid carrier or solvent, if present, then is removed from the article by, for example, drying in an oven or air drying, to provide an essentially dry coating of the enhancer material and monoester on the surface of the article. The percent dry solids on the article can range from about 4% to about 45%. As used herein, "dry coating," "essentially dry coating," "dry solids," and the like, mean that the dried article contains a dry coating having at least about 50 wt. % solids, preferably at least about 75 wt. % solids, and more preferably at least about 95 wt. % solids. Antimicrobial articles prepared according to this method are effective to kill at least 99.9% of microorganisms on the surface of the article, and preferably are effective to kill at least 99.99% of microorganisms.

The fatty acid monoester and enhancer may be most conveniently applied as essentially solventless liquid or molten compositions with the optional addition of an acceptable carrier material. For skin contact articles, suitable carrier materials include emollients and humectants such as those described in U.S. Patent No. 5,951,993. In addition, emollients such as oils (for example, hydrocarbons and alkyl esters) and skin acceptable alkyl alcohols and polyethoxylated alcohols, and combinations thereof, also may improve the skin feel of the coated articles. The molten compositions are generally applied to most articles at the lowest temperature required to keep the material suitably molten. This is typically about 50°C-150°C. The compositions are applied to most nonwoven, woven or knitted articles at a loading of 0.5 - 25 g/sq meter, more preferably 1-20 g/sq meter, and most preferably at about 2-10 g/sq meter.

Additionally, the fiber and yarn articles of this invention can be treated with the antimicrobial compositions in typical sizing-type operations. Oils and other lubricants may be added in order to ensure a good sizing.

### Characterization of Articles of the Invention

Articles of the present invention include synthetic and natural fibers or filaments. Suitable synthetic fibers, filaments, and yarns include, but are not limited to, polyolefins, polyesters, and polyamides such as nylons, polyurethanes, halogenated polyolefins, polyacrylates, polyureas, polyacrylonitriles, as well as copolymers and polymer blends. Suitable natural fibers include cotton, rayon, jute, hemp, and the like, which may be present as staple fibers or spun into yarns.

Articles of the invention also include various materials, including foam materials, nonwoven, woven, and knit fabrics or webs, fibrous batts, and sponges. The term "nonwoven web" or "nonwoven fabric" refers to a web or fabric having a structure of individual fibers that are interlaid in an irregular manner. In contrast, knit or woven fabrics have fibers that are interlaid in a regular manner. Preferable articles of the invention are made by spunbound and melt blown processes. "Spunbound" refers to small diameter fibers that are "spun" by extruding molten thermoplastic material in the form of filaments from a plurality of fine, usually circular, capillaries of a spinneret, and then rapidly reducing the diameter of the extruded filaments. See, for example, U.S. Patent Nos. 4,340,563 and 3,692,618 for a description of spunbound methods. The filaments are bonded together by passage between the rolls of a heated calender. "Melt blown" refers to a process of extruding molten thermoplastic material through a plurality of fine, typically circular, die capillaries as molten threads or filaments into a high velocity, typically heated, gas stream (e.g., air), which reduces the diameter of the filaments and deposits the filaments on a collecting surface to form a web of randomly disbursed melt blown fibers.

Non-limiting examples of particular articles include single and multi-layer nonwoven constructions and wound dressings, medical drapes and gowns, disposable diapers, filter media, face masks (e.g., surgical masks), orthopedic cast Padding/Stockinettes, textiles, respirators, food packaging, dental floss, home and industrial wipes, and battery separators.

The invention may find particular utility as an antimicrobial face mask, e.g., a surgical mask, or as an antimicrobial medical drape or gown, e.g., a surgical drape. Face masks are used as barriers between the wearer and the environment, and are well described in the art, e.g., in U.S. Pat. No. Re. 28,102 (Mayhew). Through their filtration efficiency, face masks can remove particulates (organic, inorganic, or microbiological) from the incoming or outgoing breath. Face masks are generally not antimicrobially active even though they are commonly used in a health care setting as a method of minimizing pathogen transmission risk. The invention includes a face mask with antimicrobial activity, that is a mask capable of killing microorganisms that come into contact with it. This activity extends to antimicrobial kill of such common organisms like bacteria, fungi, and viruses, e.g., the influenza A virus and the rhinovirus, the cause of the common cold. Surgical drapes may be constructed from single layers of a fibrous web material or include multi-layered laminates that include one or more film layers, e.g., as described in U.S. Pat. Nos. 3,809,077 (Hansen) and 4,522,203 (Mays). Surgical drapes require sterilization prior to use and since the drapes generally do not have inherent antimicrobial activity, any microbial contamination can remain on the surface of these drapes. The invention includes surgical drapes that can be self-sterilizing through the application of an antimicrobial coating to the surface of the surgical drape. Active surfaces like the self-sterilizing surgical drapes of this invention can provide long term antimicrobial kill of microorganisms coming in contact with the drape surface.

Antimicrobial articles of the invention may be more hydrophilic than corresponding articles not treated with a fatty acid monoester. Thus, preferred articles of the invention are capable of readily being wet with water and/or aqueous compositions. Often it is preferred that such hydrophilic articles are capable of absorbing at least 5 times their own weight with water. Absorbent, antimicrobial articles can advantageously be used as wound dressings because they are able to absorb large quantities of wound exudates and retard growth of bacteria in the absorbent layer, and, in some cases, in the wound. A further advantage of the articles is that the antimicrobial activity can reduce the sterilization load associated with the wound dressing when the device is sterilized (for example by exposure to ethylene oxide) prior to or after packaging.
The invention will be further described in the following examples, which do not limit the scope of the invention described in the claims.

### EXAMPLES

### GLOSSARY

### Monoesters

**GML:** glycerol monolaurate, available from Med-Chem Laboratories, East Lansing, Michigan under the tradename "LAURICIDIN™".
**GM-C8:** glycerol monocaprylate, available as POEM™ M-100 from Riken Vitamin LTD, Tokyo, Japan.
**GM-C10:** glycerol monocaprate, available as POEM™ M-200 from Riken Vitamin LTD, Tokyo, Japan.
**PG-C8:** propylene glycol monocaprylate, available from Uniquema International, Chicago, IL.
**PG-C10:** propylene glycol monocaprate, available from Uniquema International, Chicago, IL.

### Surfactant

**SC40:** GEMTEX™ SC40 surfactant (40% dioctyl sodium sulfosuccinate in isopropanol) commercially available from Finetex Inc., Spencer, North Carolina.

### Enhancer Materials

**LA:** Lactic acid, USP, commercially available from J.T. Baker, Phillipsburg, NJ.
**MA:** Mandelic acid, USP, commercially available from Avocado Research Chemicals, Ltd., Heysham, UK.
**SA:** Salicylic acid, USP, commercially available from J.T. Baker.

### Substrates

**PAD-1:** absorbent pads commonly used in the bottom of meat and poultry packages as absorbent pads; obtained from Cub food store, St. Paul, Minnesota.

**PAD-2:** commercially available paper (cellulose-based, available from Great Lakes Tissue Co., Cheboygan, MI) as used in multiple layers to make absorbent pads in the packaging of meat and poultry.

**PAD-3:** 100% cotton cloth.

**PAD4:** toilet paper (commercially available as 2-Ply White Tissue #0780500 Scott Surpass JRT Junior Jumbo Roll Bath Tissue from Kimberly Clark Corporation).

**MASK:** surgical masks made of nonwoven polypropylene BMF (commercially available from 3M Company, St. Paul, Minnesota, Product No. 1818).

**DRAPE-1:** Surgical drapes made of thermally laminated polyethylene film and nonwoven polypropylene BMF commercially available from 3M Company, St. Paul, Minnesota as BIOCADE™ brand surgical drapes.

**DRAPE-2:** Polypropylene BMF surgical drape commercially available from 3M Company, St. Paul, Minnesota as PREVENTION™ brand surgical drape.

### TEST METHODS

Melt-Blown Extrusion Procedure: A process similar to that described in Wente, Superfine Thermoplastic Fibers, 48 INDUS. ENG G CHEM. 1342 (1956), or in Wente et al., MANUFACTURE OF SUPERFINE ORGANIC FIBERS (Naval Research Laboratories Report No. 4364, 1954), was used for the preparation of nonwoven webs of this invention. Unless otherwise stated, the basis weight of the resulting webs was 60 ± 5 g/m² (GSM), and the desired diameter of the microfibers was 7 to 12 micrometers. The width of the web was about 12 inches (30.5 cm). Unless otherwise stated, the extrusion temperature was 255°C, the primary air temperature was 258°C, the pressure was 124 kPa (18 psi), with a 0.076 cm air gap width, and the polymer throughput rate was about 180 g/hr/cm.

Antimicrobial Test: The materials of this invention were cut into 3.8 cm x 3.8 cm square samples (unsterilized) and evaluated for antimicrobial activity according to the American Association of Textile and Color Chemists (AATCC) Test Method 100-1993, as published in the AATCC Technical Manual, 1997, Pages 143-144. Modifications to the test method included the use of Tryptic Soy Broth as the nutrient broth and for all dilutions, and Tryptic Soy Agar as the nutrient agar. Letheen Broth (VWR Scientific Products, Batavia, IL) was used as the neutralizing solution.

Water Absorbency: Evaluation of the water absorbency of certain nonwoven polypropylene articles of this invention was measured using the following test procedure. A 3.8-cm x 3.8-cm sample was weighed, dipped into 22° C deionized water for ten seconds, and then removed from the water holding a corner of the pad with the smallest possible area. The excess water was allowed to drip off from one corner until no additional water was freely dripping. The sample was then weighed again. The sample percent absorbency was then calculated using the formula: Absorbency (%) = (Wet Sample Weight - Dry Sample Weight) x 100 ö Dry Sample Weight. Results reported are the average of ten replications.

Examples 1 to 3 and Comparative Examples C1 to C3: Multiple samples of a nonwoven polypropylene BMF web (basis weight 63 g/m²) prepared using the Melt Blown Extrusion Procedure with Exxon 3866 polypropylene (Exxon Chemical Co., Baytown, TX) were dipped in an isopropanol solution of 1.0% GML and 1.0% of various enhancer materials (i.e., lactic acid (LA), salicylic acid (SA), and mandelic acid (MA)).

The samples were submerged in the isopropanol solution for approximately 10 seconds, removed, air dried, and evaluated for antimicrobial efficacy against *Staphylococcus aureus* (a Gram-positive bacteria) and against *Klebsiella pneumoniae* (a Gram-negative bacteria) using the Antimicrobial Test. The results (each data point representing a single run) are shown in Table 1. Example 3 was also evaluated for antimicrobial efficacy against *Staphylococcus epidermidis* (a Gram-positive bacteria). In each evaluation of an Example, a coated web was compared to a control of an uncoated web. Negative numbers indicate an increase from the baseline bacterial count.

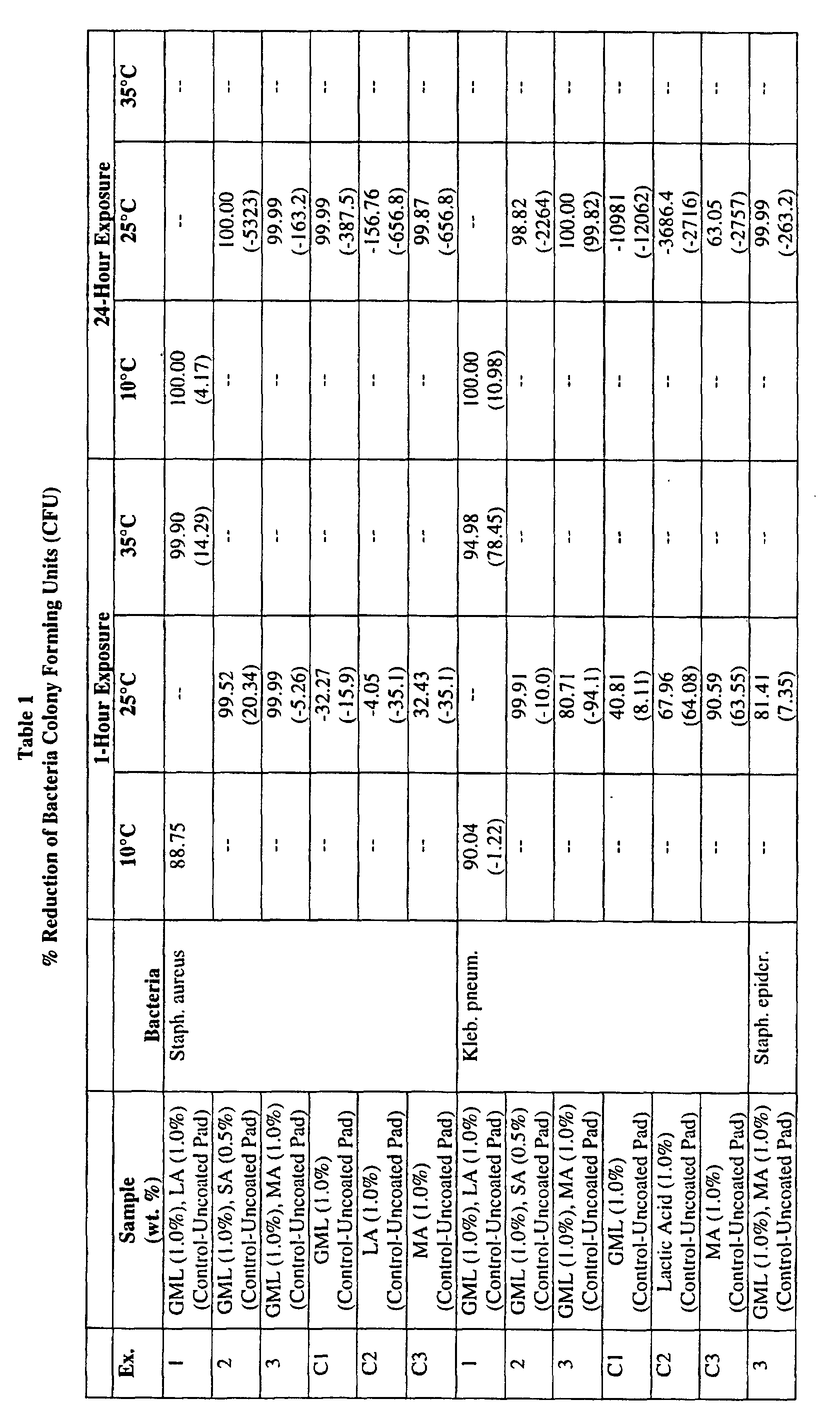

The data show that only the combination of an effective amount of GML and an enhancer material provided high antimicrobial activity to both Gram-negative and Gram-positive bacteria.

Examples 4 to 11: In these Examples, the antimicrobial efficacy of various fibrous substrates coated with GML and various enhancer materials (and optionally surfactant SC40) were evaluated. Multiple 3.8 cm x 3.8 cm samples of the coated test materials were dipped for about 10 seconds in either ethanol solutions (Examples 4 to 5), isopropanol solutions (Examples 6 to 8), or aqueous dispersions containing the SC40 surfactant (Examples 9 to 11). The samples were air dried and evaluated for antimicrobial activity according to the Antimicrobial Test using *Staphylococcus aureus* and *Klebsiella pneumoniae.* The sample materials, compositions of the solutions and aqueous dispersions, and results of the Antimicrobial Test (each data point representing a single run) are summarized in Table 2. In each instance, the treated samples were compared with an untreated control. In Examples involving the surgical mask, the entire mask was dipped into the isopropanol solution or aqueous dispersion and, after drying, 3.8 cm x 3.8 cm samples were cut for use in the Antimicrobial Test. Negative numbers indicate an increase in the bacterial count from the baseline.

The data show that all examples provided at least 99.9% control of both Gram-negative and Gram-positive bacteria when tested at 25°C to 35°C with a 24-hour exposure time. All but one sample provided at least 99.99% control of both types of bacteria at these conditions.

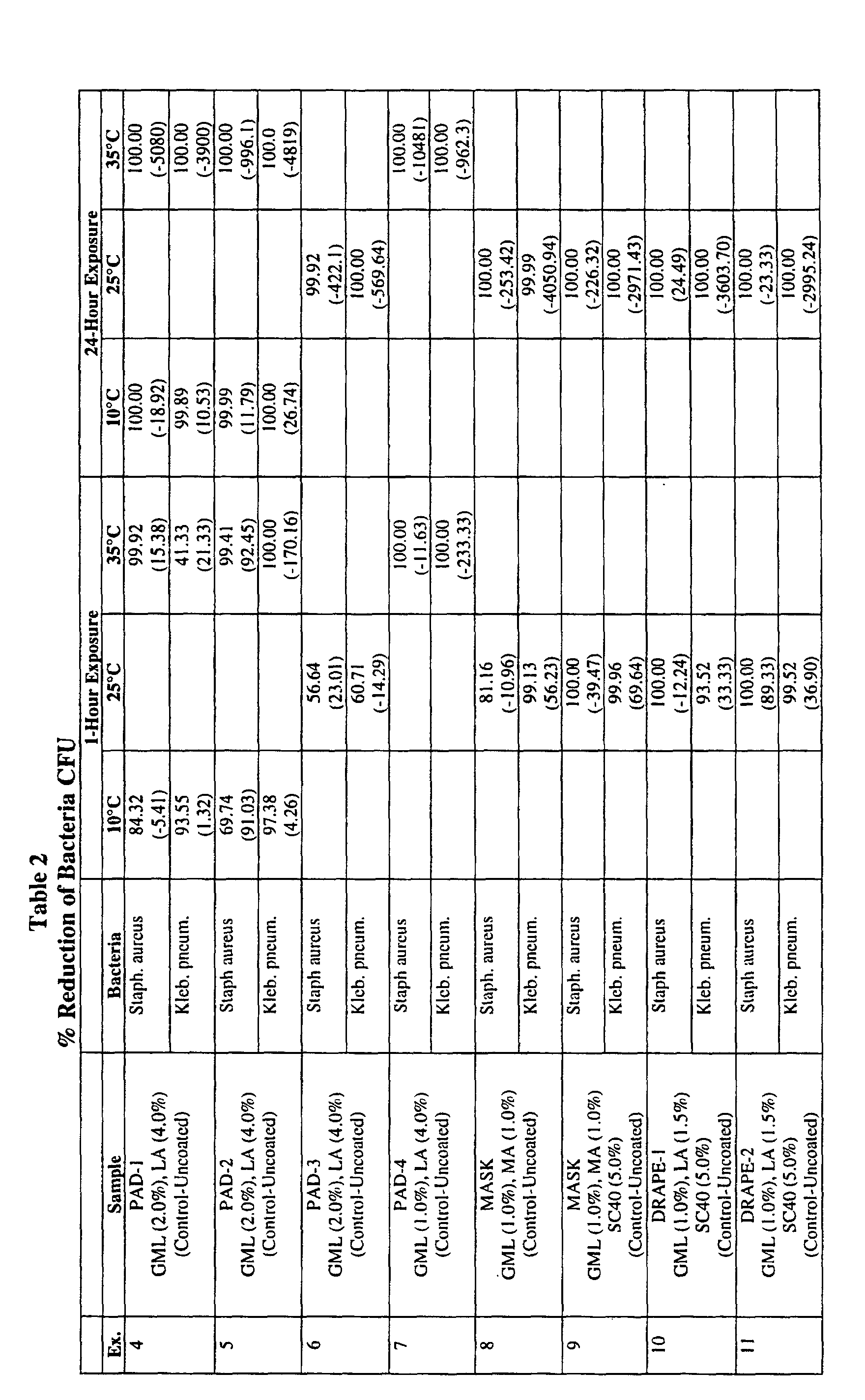

Examples 12 to 13: A 3.8-cm x 3.8-cm x 0.7-cm sample of a polyurethane open-cell foam material (POLYCRIL™ 400 with carrier and backing films removed by hand, Fulflex, Middleton, RI) was placed in a glass jar containing an isopropanol solution of 1.0% GML and 1.6% LA. The jar was capped and shaken for about 2 minutes, and the sample was removed and dried for 72 hours in a ventilation hood. The sample was weighed before and after contact with the test solution, and % Dry Solids was calculated as follows: Dry Weight (after coating) - Dry Weight (before coating) x 100 ö Dry Weight (after coating). The results (three replications) are reported in Table 3 as Example 12. A similarly coated sample of the foam material was evaluated for antimicrobial efficacy against *Staphylococcus aureus* and *Staphylococcus epidermidis* (both Gram-positive bacteria), and against *Pseudomonas aeruginosa* (a Gram-negative bacteria) using the Antimicrobial Test (test exposure times of 1 hour and 24 hours, both at 34-35° C). The results (each data point representing a single run) are also shown in Table 3. In each antimicrobial evaluation, a coated foam sample was compared to a control of an uncoated foam sample. Negative numbers indicate an increase from the baseline bacterial count.

Example 12 was repeated except that the coating composition (in place of the isopropanol solution) was an aqueous composition of GML (1.5%), LA (1.5%), SC40 surfactant (5.0%), and deionized water. The results of % Dry Solids and antimicrobial testing are provided in Table 3 as Example 13.

**Table 3**

| **% Dry Solids Coating Weight and % Reduction of Bacteria CFU** | | | | | | | | |
|---|---|---|---|---|---|---|---|---|
| Ex. | Foam Material (Coating Comp.) | % Dry Solids | *S. aureus* | | *S. epidermis* | | *P. aeruginosa* | |
| | | | 1-Hr | 24-Hr | 1-Hr | 24-Hr | 1-Hr | 24-Hr |
| 12 | GML (1.0%), LA | 13.6 | 14.02 | 98.98 | 71.56 | 100 | 44.18 | 100 |
| | (1.6%) | | (-33.6) | (-21.7) | (24.8) | (-206) | (70.6) | (-5221) |
| | (Control-Uncoated) | | | | | | | |
| 13 | GML (1.5%), LA | 42.6 | 100 | 100 | 99.18 | 99.99 | 99.24 | 99.46 |
| | (1.5%) SC40 (5.0%) | | (-33.6) | (-21.7) | (24.8) | (-206) | (70.6) | (-5221) |
| | (Control-Uncoated) | | | | | | | |

The data from Table 3 show that both coated foam samples (Examples 12 and 13) provided high antimicrobial activity (generally at least about 99% bacteria reduction, and often at least about 99.99% bacteria reduction, after 24 hours exposure at 34-35° C) to all three bacteria species.

Examples 14 to 25: Samples (3.8-cm x 3.8-cm) of a nonwoven polypropylene BMF web (basis weight 63 g/m²) prepared using the Melt Blown Extrusion Procedure with Exxon 3866 polypropylene (Exxon Chemical Co., Baytown, TX) were dipped in an isopropanol solution containing a fatty acid monoester (GML or PG-C8) and various enhancer materials (LA or MA). The samples were submerged in the isopropanol solution for approximately 10 seconds, removed, and air-dried. The samples were weighed before and after contact with the test solution, and % Dry Solids was calculated as described above. The results (ten replications) are reported in Table 4 as Examples 14-18. The samples were also evaluated for antimicrobial efficacy against *Staphylococcus aureus* (a Gram-positive bacteria) and against *Escherichia coli* (a Gram-negative bacteria) using the Antimicrobial Test (test exposure times of 1 hour and 24 hours, both at 22-23° C). The results (each data point representing a single run) are also reported in Table 4. In each antimicrobial evaluation, a coated nonwoven sample was compared to a control of an uncoated nonwoven sample. Negative numbers indicate an increase from the baseline bacterial count.

Additional experiments were conducted in the same manner as Examples 14-18, except that the coating composition (in place of the isopropanol solution) was an aqueous composition of fatty acid monoester, enhancer, SC40 surfactant, and deionized water. The results of % Dry Solids and antimicrobial testing are provided in Table 4 as Examples 19-25.

**Table 4**

| **% Dry Solids Coating Weight and % Reduction of Bacteria CFU** | | | | | | |
|---|---|---|---|---|---|---|
| **Ex.** | **Nonwoven Sample (Coating Comp.)** | **% Dry Solids** | ***S. aureus*** | | ***E. coli*** | |
| | | | **1-Hr** | **24-Hr** | **1-Hr** | **24-Hr** |
| 14 | GML (0.1%), LA (1.5%) (Control-Uncoated) | 4.9 | 85.92 (-1.97) | 100 (-8518) | 68.56 (18.73) | 64.55 (-3645) |
| 15 | GML (0.1%), MA (1.5%) (Control-Uncoated) | 9.5 | 75.68 (3.28) | 100 (-249.7) | 93.01 (25.78) | -267.2 (-4587) |
| 16 | GML (1.0%), LA (1.5%) (Control-Uncoated) | 13.5 | 95.44 (25.73) | 100 (-742.1) | 70.13 (37.34) | -2140 (-3764) |
| 17 | GML (1.0%), MA (1.5%) (Control-Uncoated) | 17.8 | 99.14 (78.61) | 100 (-229.5) | 99.99 (50.93) | 100 (-3596) |
| 18 | PG-C8 (1.0%), LA (1.5%) (Control-Uncoated) | 10.9 | 95.32 (25.73) | 99.88 (-742.1) | 99.87 (37.34) | 18.99 (-3764) |
| 19 | GML (0.1), LA (1.5%) SC40 (5.0%) (Control-Uncoated) | 20.9 | 99.93 (-1.97) | 100 (-8518) | 54.85 (18.73) | -2275 (-3645) |
| 20 | GML (0.1%), MA (1.5%) SC40 (5.0%) (Control-Uncoated) | 29.9 | 100 (3.28) | 100 (-249.7) | 99.91 (25.78) | 100 (-4587) |
| 21 | GML (0.1%), LA (3.0%) SC40 (5.0%) (Control-Uncoated) | 27.7 | 100 (25.73) | 100 (-742.1) | 72.08 (37.34) | 100 (-3764) |
| 22 | GML (1.0%), LA (1.5%) SC40 (5.0%) (Control-Uncoated) | 29.0 | 98.42 (3.28) | 100 (-249.7) | 8.59 (25.78) | -3611 (-4587) |
| 23 | GML (1.0%), LA (3.0%) SC40 (5.0%) (Control-Uncoated) | 28.6 | 99.99 (78.61) | 100 (-229.5) | 78.26 (50.93) | -1794 (-3596) |
| 24 | GML (1.0%), MA (1.5%) SC40 (5.0%) (Control-Uncoated) | 34.3 | 100 (78.61) | 100 (-229.5) | 99.97 (50.93) | 100 (-3596) |
| 25 | PG-C8 (1.0%), LA (1.5%) SC40 (5.0%) (Control-Uncoated) | 25.8 | 99.98 (25.73) | 100 (-742.1) | 77.92 (37.34) | -1247 (-3764) |

The data from Table 4 show that all of the coated nonwoven samples (Examples 14-25) provided high antimicrobial activity (generally at least about 99.9% bacteria reduction, after 24 hours exposure at 22-23° C) to at least one of the two bacteria species. Several samples (e.g., Examples 17, 20, 21, and 24) provided 100% reduction of both *S. aureus* and *E. coli* bacteria.

Examples 26 to 40: Samples (3.8-cm x 3.8-cm) of a nonwoven polypropylene BMF web (basis weight 63 g/m²) prepared using the Melt Blown Extrusion Procedure with Exxon 3866 polypropylene were coated with a fatty acid monoester and an enhancer material as described in Examples 14-25. Certain samples (Examples 26-31) were dipped in isopropanol solutions and other samples (Examples 32-40) were dipped in an aqueous composition that included the SC40 surfactant. All samples were evaluated for antimicrobial efficacy against *Staphylococcus aureus* and against *Escherichia coli* using the Antimicrobial Test, and % Dry Solids were calculated. The results (each data point representing a single run) are provided in Table 5. In each antimicrobial evaluation, a coated nonwoven sample was compared to a control of an uncoated nonwoven sample. Negative numbers indicate an increase from the baseline bacterial count.

**Table 5**

| **% Dry Solids Coating Weight and % Reduction of Bacteria Colony Forming Units (CFU)** | | | | | | |
|---|---|---|---|---|---|---|
| Ex. | Foam Sample (Coating Comp.) | % Dry Solids | *S. aureus* | | *E. coli* | |
| | | | 1-Hr | 24-Hr | 1-Hr | 24-Hr |
| 26 | GML (0.1%), LA (3.0%)(Control - Uncoated) | 9.4 | 92.18 (21.33) | 100 (-833.3) | -202.6 (18.95) | -1374 (44.74) |
| 27 | GML (1.0%), MA (3.0%) (Control - Uncoated) | 25.8 | 99.10 (21.33) | 100 (-833.3) | 99.67 (18.95) | 100 (44.74) |
| 28 | GML (0.1%), MA (3.0%) (Control - Uncoated) | 21.7 | 100 (17.27) | 100 (-1394) | 100 (41.69) | 100 (-6239) |
| 29 | PG-C8 (1.0%), MA (3.0%) (Control - Uncoated) | 23.8 | 100 (17.27) | 100 (-1394) | 100 (41.69) | 100 (-6239) |
| 30 | PG-C8 (1.0%), MA (1.5%) (Control - Uncoated) | 17.3 | 100 (17.27) | 100 (-1394) | 100 (41.69) | 98.90 (-6239) |
| 31 | PG-C8 (1.0%), LA (3.0%) (Control - Uncoated) | 19.6 | 99.99 (9.27) | 100 (-818.9) | 100 (-9.90) | 100 (-4431) |
| 32 | GML (1.0%), MA (3.0%) SC40 (5.0%) (Control - Uncoated) | 38.1 | 100 (21.33) | 100 (-833.3) | 97.00 (18.95) | 100 (44.74) |
| 33 | GML (0.1%), MA (3.0%) SC40 (5.0%) (Control - Uncoated) | 34.3 | 100 (21.33) | 100 (-833.3) | 100 (18.95) | 100 (44.74) |
| 34 | GML (0.1%), MA (0.25%) SC40 (5.0%) (Control - Uncoated) | 19.5 | 99.64 (17.27) | 100 (-1394) | 52.20 (41.69) | -1815 (-6239) |
| 35 | PG-C8 (1.0%), MA (1.5%) SC40 (5.0%) (Control - Uncoated) | 31.5 | 100 (17.27) | 100 (-1394) | 100 (41.69) | 100 (-6239) |
| 36 | PG-C8 (1.0%), MA (3.0%) SC40 (5.0%) (Control - Uncoated) | 38.6 | 100 (9.27) | 100 (-818.9) | 100 (-9.90) | 100 (-4431) |
| 37 | PG-C8 (1.0%), LA (3.0%) SC40 (5.0%) (Control - Uncoated) | 27.2 | 100 (9.27) | 100 (-818.9) | 90.49 (9.90) | 100 (-4431) |
| 38 | GM-C8 (1.0%, LA (1.5%) SC40 (5.0%) (Control - Uncoated) | 33.1 | 99.95 (9.27) | 100 (-818.9) | 96.74 (-9.90) | 100 (-4431) |

| Ex. | Nonwoven Sample (Coating Comp.) | % Dry Solids | S. aureus | | E. coli | |
|---|---|---|---|---|---|---|
| | | | 1-Hr | 24-Hr | 1-Hr | 24-Hr |
| 39 | PG-C 10 ( 1.0%), LA (1.5%) SC40 (5.0%) (Control - Uncoated) | 24.9 | 97.97 (9.27) | 99.98 (-818.9) | 52.08 (-9.90) | -2582 (-4431) |
| 40 | GM-C10 (1.0%), LA (1.5%) SC40 (5.0%) (Control - Uncoated) | 28.1 | 99.95 (9.27) | 100 (-818.9) | 94.90 (-9.90) | -1202 (-4431) |

The data from Table 5 show that all of the coated nonwoven samples (Examples 26-40) provided high antimicrobial activity (generally at least about 99.9% bacteria reduction, after 24 hours exposure at 22-23° C) to at least one of the two bacteria species. All but one sample (Example 39) provided 100% reduction of *Staphylococcus aureus* bacteria and all but five samples (Examples 26, 30, 34, 39, and 40) provided 100% reduction of *Escherichia coli* bacteria at these conditions.

Example 41: This Example illustrates the degree of water absorbency of the treated nonwoven web constructions of this invention as compared to an untreated control web.

A sample (3.8-cm x 3.8-cm) of a nonwoven polypropylene BMF web (basis weight 63 g/m²) prepared using the Melt Blown Extrusion Procedure with Exxon 3866 polypropylene was treated with an aqueous composition of GML (1.0%), LA (1.5%), SC40 surfactant (5.0%), and deionized water as described in Examples 19-25.

The amount of water absorbed and the percent water absorbency of the sample (Example 41) was measured according to the Water Absorbency Method described above. Results are provided in Table 6 and are compared to the results of testing an identical nonwoven sample that had not been treated. These data show that the treated sample was highly water absorbent, absorbed over eight times its own weight with water, and absorbed over ten times as much water as the untreated sample.

**Table 6**

| Example | Components of Treatment Composition | Dry Sample Weight (g) | Water Absorbed (g) | Water Absorbency (%) |
|---|---|---|---|---|
| 41 | GML (1.0%), LA (1.5%), SC-40 (5.0%) | 0.150 | 1.22 | 813 |
| Control | None | 0.105 | 0.114 | 108 |

## Claims

1. An antimicrobial article comprising a coating, said coating comprising a fatty acid monoester and an enhancer, said enhancer being a chelating agent or an organic acid, wherein said fatty acid monoester and said enhancer are present in an amount effective to kill at least 99.9% of microorganisms.

2. The antimicrobial article of claim 1, wherein said fatty acid monoester is glycerol monolaurate, glycerol monocaprylate, glycerol monocaprate, propylene glycol monolaurate, propylene glycol monocaprylate, and propylene glycol monocaprate.

3. The antimicrobial article of claim 1, wherein said organic acid is selected from the group consisting of lactic acid, tartaric acid, adipic acid, succinic acid, citric acid, ascorbic acid, malic acid, mandelic acid, acetic acid, sorbic acid, benzoic acid, and salicylic acid.

4. The antimicrobial article of claim 1, wherein said organic acid is lactic acid or mandelic acid.

5. The antimicrobial article of claim 1, wherein said chelating agent is sodium acid pyrophosphate, acidic sodium hexametaphosphate, or ethylenediaminetetraacetic acid and salts thereof.

6. The antimicrobial article of claim 1, wherein said article is a nonwoven material.

7. The antimicrobial article of claim 1, wherein said article is a woven fabric or web material, a sponge material, a fibrous batt material, or a foam material.

8. The antimicrobial article of claim 1, wherein said coating further comprises a surfactant.

9. The antimicrobial article of claim 1, wherein said microorganisms comprise Gram positive or Gram negative bacteria.

10. The antimicrobial article of claim 1, wherein said microorganisms comprise viruses or fungi.

11. The antimicrobial article of claim 1, wherein said fatty acid monoester and said enhancer are present in an amount effective to kill at least 99.99% of microorganisms.

12. The antimicrobial article of claim 1, wherein said article has increased hydrophilicity as compared with a corresponding article lacking said fatty acid monoester.

13. The antimicrobial article of claim 1, wherein said article is a surgical drape or gown, a face mask, a wound dressing, a disposable diaper, filter media, a cast padding, a stockinette, a respirator, food packaging, dental floss, a sponge, textiles, or a wipe.

14. A method of making an antimicrobial article, said method comprising treating said article with an antimicrobial composition, wherein said composition comprises 0.001 wt. % to 30 wt. % of a fatty acid monoester and 0.001 wt. % to 85 wt. % of an enhancer, said enhancer being a chelating agent or an organic acid, and drying said treated article, wherein said antimicrobial article is effective for killing at least 99.9% of microorganisms.

15. The method of claim 14, wherein said fatty acid monoester comprises 0.01 wt. % to 5.0 wt. % of said antimicrobial composition.

16. The method of claim 14, wherein said enhancer comprises 0.5 wt. % to 5.0 wt. % of said antimicrobial composition.

17. The method of claim 14, wherein said antimicrobial composition further comprises 0.001 wt. % to 30 wt. % of a surfactant.

18. The method of claim 17, wherein said surfactant comprises 0.5 wt. % to 5.0 wt. % of said antimicrobial composition.

19. A method of making an antimicrobial article, said method comprising treating said article with a first composition comprising 0.001 wt. % to 30 wt. % of a fatty acid monoester; treating said article with a second composition comprising 0.001 wt. % to 85 wt. % of an enhancer, said enhancer being a chelating agent or an organic acid, and drying said treated article, wherein said antimicrobial article is effective for killing at least 99.9% of microorganisms.

20. The method of claim 19, wherein said fatty acid monoester comprises 0.01 wt. % to 5.0 wt. % of said first composition.

21. The method of claim 19, wherein said enhancer comprises 0.5 wt. % to 5.0 wt. % of said second composition.

22. The method of claim 19, wherein said first composition further comprises 0.001 wt. % to 30 wt. % of a surfactant.

23. The method of claim 22, wherein said surfactant comprises 0.5 wt. % to 5.0 wt. % of said first composition.

## Patentansprüche

1. Antimikrobieller Gegenstand, umfassend eine Beschichtung, wobei die Beschichtung einen Fettsäuremonoester und einen Verstärker umfasst, wobei der Verstärker ein chelatisierendes Mittel oder eine organische Säure ist, wobei der Fettsäuremonoester und der Verstärker in einer Menge vorhanden sind, die wirksam ist, um mindestens 99,9% der Mikroorganismen zu töten.

2. Antimikrobieller Gegenstand gemäß Anspruch 1, wobei der Fettsäuremonoester Glycerinmonolaurat, Glycerinmonocaprylat, Glycerinmonocaprinat, Propylenglycolmonolaurat, Propylenglycolmonocaprylat und Propylenglycolmonocaprinat ist.

3. Antimikrobieller Gegenstand gemäß Anspruch 1, wobei die organische Säure aus Milchsäure, Weinsäure, Adipinsäure, Bernsteinsäure, Zitronensäure, Ascorbinsäure, Äpfelsäure, Mandelsäure, Essigsäure, Sorbinsäure, Benzoesäure und Salicylsäure ausgewählt ist.

4. Antimikrobieller Gegenstand gemäß Anspruch 1, wobei die organische Säure Milchsäure oder Mandelsäure ist.

5. Antimikrobieller Gegenstand gemäß Anspruch 1, wobei das chelatisierende Mittel Natriumsäurepyrophosphat, Natriumhexametaphosphat oder Ethylendiamintetraessigsäure und Salze davon ist.

6. Antimikrobieller Gegenstand gemäß Anspruch 1, wobei der Gegenstand ein Vliesmaterial ist.

7. Antimikrobieller Gegenstand gemäß Anspruch 1, wobei der Gegenstand ein Textilgewebe oder Bahnenmaterial, ein Schwammmaterial, ein Fasermaterial ("fibrous batt") oder ein Schaumstoff ist.

8. Antimikrobieller Gegenstand gemäß Anspruch 1, wobei die Beschichtung weiterhin ein oberflächenaktives Mittel umfasst.

9. Antimikrobieller Gegenstand gemäß Anspruch 1, wobei die Mikroorganismen Gram-positive oder Gram-negative Bakterien umfassen.

10. Antimikrobieller Gegenstand gemäß Anspruch 1, wobei die Mikroorganismen Viren oder Pilze umfassen.

11. Antimikrobieller Gegenstand gemäß Anspruch 1, wobei der Fettsäuremonoester und der Verstärker in einer Menge anwesend sind, die wirksam ist, um mindestens 99,99% der Mikroorganismen zu töten.

12. Antimikrobieller Gegenstand gemäß Anspruch 1, wobei der Gegenstand eine erhöhte Hydrophilie aufweist verglichen mit einem entsprechenden Gegenstand, der den Fettsäuremonoester nicht enthält.

13. Antimikrobieller Gegenstand gemäß Anspruch 1, wobei der Gegenstand ein Chirurgie-Tuch oder Chirurgiekittel, eine Gesichtsmaske, ein Wundverband, eine Wegwerfwindel, Filtermedium, eine Gipspolsterung, eine Stockinette, eine Atemschutzmaske, Nahrungsmittelverpackung, Zahnseide, ein Schwamm, Textilien oder ein Wischgegenstand ist.

14. Verfahren zur Herstellung eines antimikrobiellen Gegenstands, wobei das Verfahren umfasst: Behandeln des Gegenstands mit einer antimikrobiellen Zusammensetzung, wobei die Zusammensetzung 0,001 Gew.-% bis 30 Gew.-% eines Fettsäuremonoesters und 0,001 Gew.-% bis 85 Gew.-% eines Verstärkers umfasst, wobei der Verstärker ein chelatisierendes Mittel oder eine organische Säure ist, und Trocknen des behandelten Gegenstands, wobei der antimikrobielle Gegenstand wirksam ist, um mindestens 99,9% der Mikroorganismen zu töten.

15. Verfahren gemäß Anspruch 14, wobei der Fettsäuremonoester 0,01 Gew.-% bis 5,0 Gew.-% der antimikrobiellen Zusammensetzung umfasst.

16. Verfahren gemäß Anspruch 14, wobei der Verstärker 0,5 Gew.-% bis 5,0 Gew.-% der antimikrobiellen Zusammensetzung umfasst.

17. Verfahren gemäß Anspruch 14, wobei die antimikrobielle Zusammensetzung weiterhin 0,001 Gew.-% bis 30 Gew.-% eines oberflächenaktiven Mittels umfasst.

18. Verfahren gemäß Anspruch 17, wobei das oberflächenaktive Mittel 0,5 Gew.-% bis 5,0 Gew.-% der antimikrobiellen Zusammensetzung umfasst.

19. Verfahren zur Herstellung eines antimikrobiellen Gegenstands, wobei das Verfahren umfasst: Behandeln des Gegenstands mit einer ersten Zusammensetzung, umfassend 0,001 Gew.-% bis 30 Gew.-% eines Fettsäuremonoesters; Behandeln des Gegenstands mit einer zweiten Zusammensetzung, umfassend 0,001 Gew.-% bis 85 Gew.-% eines Verstärkers, wobei der Verstärker ein chelatisierendes Mittel oder eine organische Säure ist, und Trocknen des behandelten Gegenstands, wobei der antimikrobielle Gegenstand wirksam ist, um mindestens 99,9% der Mikroorganismen zu töten.

20. Verfahren gemäß Anspruch 19, wobei der Fettsäuremonoester 0,01 Gew.-% bis 5,0 Gew.-% der ersten Zusammensetzung umfasst.

21. Verfahren gemäß Anspruch 19, wobei der Verstärker 0,5 Gew.-% bis 5,0 Gew.-% der zweiten Zusammensetzung umfasst.

22. Verfahren gemäß Anspruch 19, wobei die erste Zusammensetzung weiterhin 0,001 Gew.-% bis 30 Gew.-% eines oberflächenaktiven Mittels umfasst.

23. Verfahren gemäß Anspruch 22, wobei das oberflächenaktive Mittel 0,5 Gew.-% bis 5,0 Gew.-% der ersten Zusammensetzung umfasst.

## Revendications

1. Article antimicrobien comprenant un revêtement, ledit revêtement comprenant un monoester d'acide gras et un amplificateur, ledit amplificateur étant un agent chélatant ou un acide organique, dans lequel ledit monoester d'acide gras et ledit amplificateur sont présents en une quantité efficace pour tuer au moins 99,9 % de microorganismes.

2. Article antimicrobien selon la revendication 1, dans lequel ledit monoester d'acide gras est le glycérol monolaurate, le glycérol monocaprylate, le glycérol monocaprate, le propylène glycol monolaurate, le propylène glycol monocaprylate et le propylène glycol monocaprate.

3. Article antimicrobien selon la revendication 1, dans lequel ledit acide organique est choisi dans le groupe constitué par l'acide lactique, l'acide tartrique, l'acide adipique, l'acide succinique, l'acide citrique, l'acide ascorbique, l'acide malique, l'acide mandélique, l'acide acétique, l'acide sorbique, l'acide benzoïque et l'acide salicylique.

4. Article antimicrobien selon la revendication 1, dans lequel ledit acide organique est l'acide lactique ou l'acide mandelique.

5. Article antimicrobien selon la revendication 1, dans lequel ledit agent chélatant est le pyrophosphate acide de sodium, l'hexamétaphosphate de sodium acide ou l'acide éthylènediaminetétraacétique et leurs sels.

6. Article antimicrobien selon la revendication 1, dans lequel ledit article est une matière non tissée.

7. Article antimicrobien selon la revendication 1, dans lequel ledit article est un tissu tissé ou une matière en toile, une matière en éponge, une matière en nappage fibreux (fibrous batt) ou une matière en mousse.

8. Article antimicrobien selon la revendication 1, dans lequel ledit revêtement comprend en outre un surfactant.

9. Article antimicrobien selon la revendication 1, dans lequel lesdits microorganismes comprennent les bactéries Gram positives ou Gram négatives.

10. Article antimicrobien selon la revendication 1, dans lequel lesdits microorganismes comprennent les virus ou les champignons.

11. Article antimicrobien selon la revendication 1, dans lequel ledit monoester d'acide gras et ledit amplificateur sont présents en une quantité efficace pour tuer au moins 99,99 % de microorganismes.

12. Article antimicrobien selon la revendication 1, dans lequel ledit article a une hydrophilicité accrue en comparaison avec un article correspondant auquel il manque ledit monoester d'acide gras.

13. Article antimicrobien selon la revendication 1, dans lequel ledit article est un drap opératoire ou une blouse chirurgicale, un masque facial, un pansement, une couche jetable, une média filtrante, un rembourrage de plâtre, une stockinette, un masque filtrant, un emballage alimentaire, du fil dentaire, une éponge, des textiles ou un essuie-tout.

14. Procédé de réalisation d'un article antimicrobien, ledit procédé comprenant le traitement dudit article avec une composition antimicrobienne, dans lequel ladite composition comprend de 0,001 % en poids à 30 % en poids d'un monoester d'acide gras et de 0,001 % en poids à 85 % en poids d'un amplificateur, ledit amplificateur étant un agent chélatant ou un acide organique, et le séchage dudit article traité, dans lequel ledit article antimicrobien est efficace pour tuer au moins 99,9 % de microorganismes.

15. Procédé selon la revendication 14, dans lequel ledit monoester d'acide gras comprend de 0,01 % en poids à 5,0 % en poids de ladite composition antimicrobienne.

16. Procédé selon la revendication 14, dans lequel ledit amplificateur comprend de 0,5 % en poids à 5,0 % en poids de ladite composition antimicrobienne.

17. Procédé selon la revendication 14, dans lequel ladite composition antimicrobienne comprend en outre de 0,001 % en poids à 30 % en poids d'un surfactant.

18. Procédé selon la revendication 17, dans lequel ledit surfactant comprend de 0,5 % en poids à 5,0 % en poids de ladite composition antimicrobienne.

19. Procédé de réalisation d'un article antimicrobien, ledit procédé comprenant le traitement dudit article avec une première composition comprenant de 0,001 % en poids à 30 % en poids d'un monoester d'acide gras ; le traitement dudit article avec une seconde composition comprenant de 0,001 % en poids à 85 % en poids d'un amplificateur, ledit amplificateur étant un agent chélatant ou un acide organique, et le séchage dudit article traité, dans lequel ledit article antimicrobien est efficace pour tuer au moins 99,9 % de microorganismes.

20. Procédé selon la revendication 19, dans lequel ledit monoester d'acide gras comprend de 0,01 % en poids à 5,0 % en poids de ladite première composition.

21. Procédé selon la revendication 19, dans lequel ledit amplificateur comprend de 0,5 % en poids à 5,0 % en poids de ladite seconde composition.

22. Procédé selon la revendication 19, dans lequel ladite première composition comprend en outre de 0,001 % en poids à 30 % en poids d'un surfactant.

23. Procédé selon la revendication 22, dans lequel ledit surfactant comprend de 0,5 % en poids à 5,0 % en poids de ladite première composition.
